# EUROPÄISCHE PATENTSCHRIFT

(11) **EP 1 304 076 B1**
(45) Veröffentlichungstag und Bekanntmachung des Hinweises auf die Patenterteilung: **23.02.2005**
(21) Anmeldenummer: 02020370.9
(22) Anmeldetag: 12.09.2002
(51) Int. Cl.: A61B 6/04, G03B 42/02, A61B 6/00

(54) **Röntgendiagnostikgerät für Mammographieuntersuchungen**
X-ray diagnosis apparatus for mammography
Appareil radiodiagnostique pour la mammographie

(30) Priorität: 18.10.2001 SE 0103474
(43) Veröffentlichungstag der Anmeldung: 23.04.2003
(73) Patentinhaber: Siemens Aktiengesellschaft, 80333 München (DE)
(72) Erfinder: Andreasson, Jesper, 177 41 Järfälla (SE); Hoff, Ann-Sofi, 112 54 Stockholm (SE); Karlsson, Stefan, 191 34 Sollentuna (SE)

(56) Entgegenhaltungen:
- EP-A- 0 446 102
- WO-A-88/01847
- US-A- 4 989 227

## Beschreibung

Die Erfindung betrifft ein Röntgendiagnostikgerät für Mammographieuntersuchungen mit einem Arm für eine Röntgenröhre, einer Halterung für einen Bildempfänger und einem Objekttisch, wobei der Objekttisch seitlich gegen die Halterung verschiebbar ist, sowie eine zwischen der Röntgenröhre und dem Objekttisch angeordnete Kompressionsplatte, wobei der Arm um eine in einem Stativ angebrachte horizontale Welle drehbar angeordnet ist und die Halterung für den Bildempfänger mit dem Arm verbunden ist.

Aus der WO 88/ 01847 A1 ist eine Vorrichtung zur lateralen Verschiebung des Objekttisches mit der zwischen Kompressionsplatte und Objekttisch eingespannten Brust bekannt. Die Vorrichtung hat den Zweck, durch Röntgenaufnahmen aus zwei Richtungen die Position verdächtiger Knoten innerhalb der Brust mittels einfacher Geometrie zu bestimmen.

Bei einer Mammographieuntersuchung ist es von Vorteil, wenn die Größe des Bildempfängers an die Grosse des Objekttisches angepasst ist. In den Fällen, in denen der Bildempfänger ein in einer Kassette angebrachter Röntgenfilm ist, gibt es auf dem Markt zwei Kassettengrößen, wobei die größere Röntgenfilmkassette die Maße 24 x 30 cm und die kleinere Röntgenfilmkassette die Maße 18 x 24 cm aufweist.

Eine Möglichkeit, die praktiziert und in der US-PS 4 613 982 beschrieben wird, ist es, einen Objekttisch für jede Kassettengröße zu haben, wobei die Breite des Objekttisches der Breite der Kassette entspricht. Auf diese Weise kann der Benutzer, abhängig von der Größe des Objekts, zwischen den Kassettengrößen wechseln. Mehrere Objekttische zu haben ist verhältnismäßig teuer und ein Wechseln des Objekttisches kann zeitraubend sein.

Der Grund, weshalb die Breite der Kassette bzw. des Röntgenfilms dieselbe wie die Breite des Objekttisches sein sollte ist, dass bei einer seitlichen Aufnahme der Objekttisch und damit auch der Röntgenfilm in die Achselhöhle des Patienten verschoben werden kann, damit so viel Brustgewebe wie möglich abgebildet wird.

In Verbindung mit einem Röntgendiagnostikgerät der Eingangs genannten Art, das durch die US-PS 4 989 227 bekannt ist, ist ein Versuch gemacht worden, die Mammographieuntersuchung zu vereinfachen und die Kosten zu verringern, indem lediglich ein Objekttisch für die größenmäßig unterschiedlichen Röntgenfilmkassetten verwendet wird. Der Objekttisch, der an dem Arm für die Röntgenröhre befestigt ist, weist eine Breite auf, die der Breite der größeren der beiden Kassetten entspricht. Bei einer seitlichen Aufnahme ist daher mit einer im Objekttisch vorhandenen größeren Röntgenfilmkassette eine gewünschte Abbildung des Brustgewebes gegeben.

Bei der Verwendung der kleineren Kassette kann diese mit Hilfe von den in der erwähnten US-Patentschrift beschriebenen Mitteln im Objekttisch in drei festen Lagen, teils in eine zentrierte Lage, in der die Kassette mit Abstand zu den Endseiten des Tisches angebracht ist, teils in eine Lage, in der die eine Endseite der Kassette entlang der einen Seite des Objekttisches liegt und teils in eine Lage, in der die andere Endseite der Kassette entlang der anderen Seite des Objekttisches liegt, verschoben werden.

Bei einer vertikalen Aufnahme der Brust ist die Kassette in einer beschriebenen zentrierten Lage angebracht. Bei einer seitlichen Aufnahme wird der Objekttisch mit Hilfe des bereits beschriebenen Armes um die horizontale Welle so gedreht, dass der Objekttisch in die Achselhöhle des Patienten hingeschoben wird. In dieser Lage wird die Kassette im Objekttisch in eine Lage verschoben, in der die oberen Endseite der Kassette entlang der oberen Seite des Objekttisches liegt. In Verbindung mit einer Verschiebung der Kassette im Objekttisch deckt das Strahlenfeld der Röntgenröhre nicht mehr die ganze Oberfläche der Kassette bzw. der Röntgenröhre. Bei einer seitlichen Aufnahme muss daher eine Einstellung der Blenden der Röntgenröhre erneut vorgenommen werden, was relativ zeitraubend ist. Das kann für den Patienten unangenehm und zum Teil schmerzhaft sein, da die Brust zwischen dem Objekttisch und der Kompressionsplatte eingespannt ist.

Der Erfindung liegt die Aufgabe zurunde, ein Röntgendiagnostikgerät der Eingangs genannten Art zu schaffen, bei dem beim Drehen des Objekttisches bzw. des Bildempfängers von einer vertikalen Lage in eine Seitenlage der Bildempfänger mittels einfacher und dadurch billiger Mittel im Röntgenstrahlenfeld liegenbleiben kann.

Diese Aufgabe wird erfindungsgemäß dadurch gelöst, dass die Kompressionsplatte mit dem Arm verbunden und entlang diesem verschiebbar und der Objekttisch so ausgebildet ist, dass er gegenüber der Halterung und gegenüber dem Bildempfänger aus einer Ausgangsposition seitlich in eine erste und eine zweite Endposition verschiebbar ist, wobei die Ausgangsposition derart ist, dass beide Kurzseiten des Bildempfängers in gleicher Entfernung von den Seiten des Objekttisches angeordnet sind und die Endpositionen derart sind, dass jeweils eine Kurzseite des Bildempfängers im wesentlichen mit der Seite des Objekttisches abschließt. In Verbindung mit einer seitlichen Aufnahme d.h., wenn der Bildempfänger in die Achselhöhle des Patienten hineingeschoben wird, wird der Objekttisch in die jeweilige Endposition verschoben, in der der Bildempfänger im Röntgenstrahlfeld verbleibt. Eine Aufnahme kann deshalb nun verhältnismäßig schnell vorgenommen werden. Da sowohl Objekttisch als auch Bildempfänger in die Achselhöhle des Patienten verschoben sind wird so viel Brustgewebe wie möglich abgebildet.

Die Erfindung ist nachfolgend anhand eines in den Zeichnungen dargestellten Ausführungsbeispiels näher erläutert. Es zeigen:
- FIG 1: eine Seitenansicht eines Röntgendiagnostikgerätes für Mammographieuntersuchungen nach der Erfindung;
- FIG 2: eine Draufsicht eines Objekttisches des Röntgendiagnostikgerätes nach Fig. 1 und
- FIG 3: eine weitere Draufsicht eines Objekttisches gemäß Fig. 2

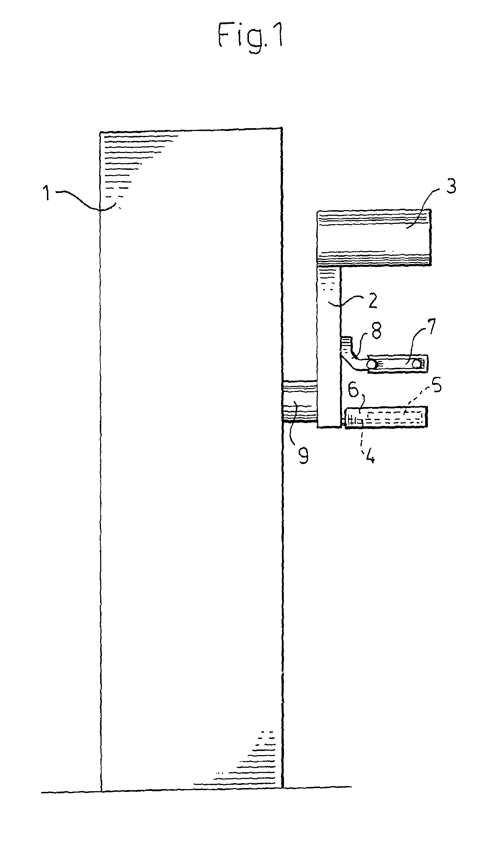

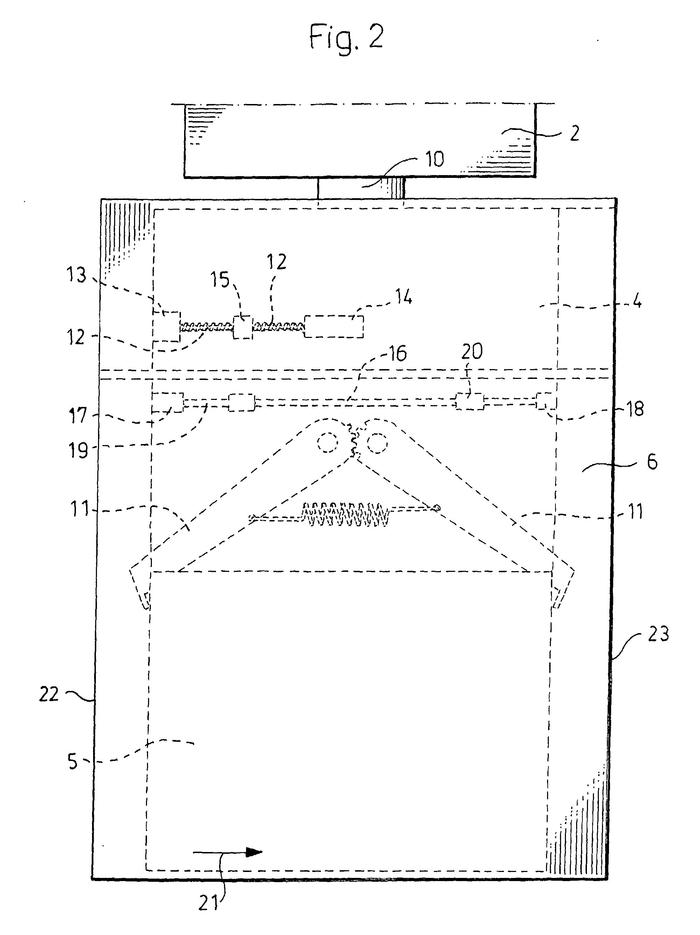

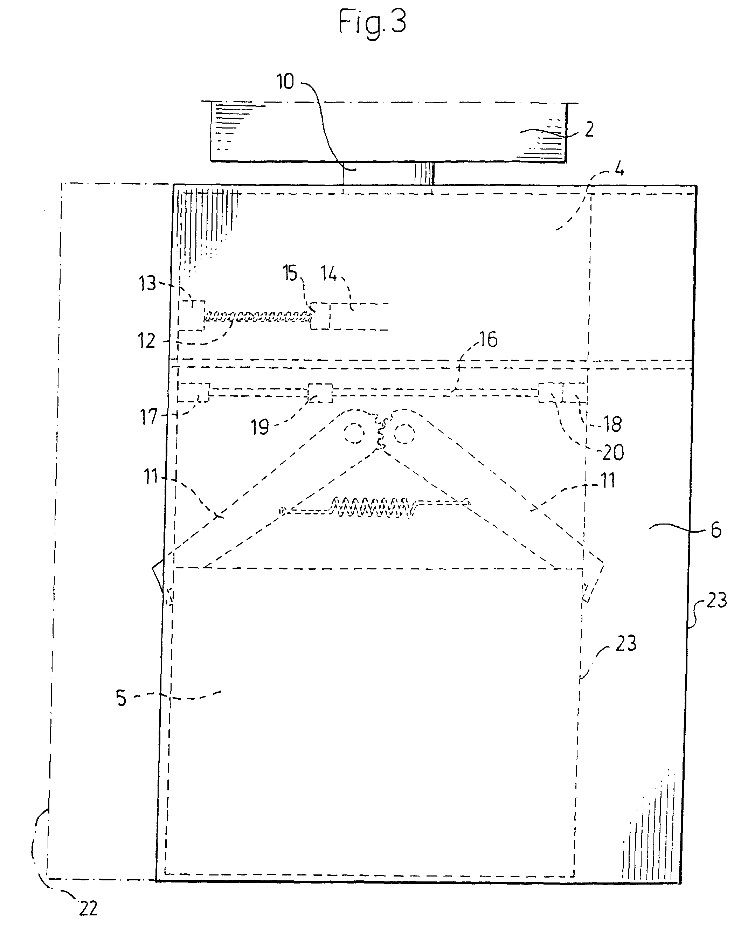

In der Fig. 1 ist ein Röntgendiagnostikgerät für Mammographieuntersuchungen mit einem Stativ 1, das einen Arm 2 für eine Röntgenröhre 3 trägt, schematisch gezeigt. Der Arm 2 trägt ferner eine Halterung 4 für einen Bildempfänger 5 sowie einen Objekttisch 6, wobei die Halterung 4 bzw. der Objekttisch 6 in Verbindung mit den Fig. 2 und 3 näher beschrieben werden. Zwischen der Röntgenröhrer 3 und dem Objekttisch 6 ist eine Kompressionsplatte 7 angeordnet, die über eine Konsole 8 mit dem Arm 2 entlang diesem verschiebbar verbunden ist. Der Arm 2 ist über eine horizontale Welle 9 mit dem Stativ 1 drehbar verbunden.

In der Fig. 2 ist in einer Draufsicht gezeigt, dass die Halterung 4 des Bildempfängers 5 mit dem Arm 2 über eine Welle 10 verbunden ist. Der Bildempfänger 5 ist in diesem Ausführungsbeispiel eine Kassette für einen Röntgenfilm. Die in der Figur gezeigte Röntgenfilmkassette 5 ist die kleinere von den in der Einleitung beschriebenen Kassetten.

Die Halterung 4 ist vorzugsweise eine Platte mit einer Breite, die der Breite der kleineren der beiden Kassetten entspricht. Die Kassette 5 ist an der Halterung 4 mittels zweier, an der Halterung 4 angeordneter federgespannter Greifarme 11 festgespannt. Die Halterung 4 ist mit einer Schraube 12 versehen, die in der Querrichtung der Halterung 4 angeordnet ist, wobei das eine Ende 13 der Schraube 12 an der Halterung 4 und dessen andere Ende mit einem Motor 14 verbunden ist. Die Schraube 12 ist ferner mit einer mit Gewinde versehenen Hülse 15, die ihrerseits am Tisch befestigt ist, verbunden.

Die Halterung 4 ist ausserdem mit einer Stange 16 versehen, die parallel zur Schraube 12 angeordnet ist und die entlang der Breite der Halterung 4 verläuft, wobei die Enden der Stange 16 über Anschläge 17, 18 mit der Halterung 4 fest verbunden sind. Ferner sind Hülsen 19, 20 vorgesehen, die entlang der Stange 16 verschiebbar angeordnet sind, wobei die Hülsen am Objekttisch 6 befestigt sind.

Bei einer vertikalen Aufnahme einer Brust eines Patienten wird die Brust auf den Objekttisch placiert. Danach wird die Brust mit Hilfe der Kompressionsplatte 7 zusammengepresst, woraufhin eine Aufnahme vorgenommen wird. In Verbindung mit einer Drehung der Halterung 4 bzw. des Objekttisches 6 mit Hilfe des Armes 2 um die Welle 9 in eine Seitenlage für eine seit-liche Aufnahme z.B. von der linken Achselhöhle des Patienten, wird der Objekttisch 6 gegenüber der Halterung 4 in die Richtung des Pfeiles 21 so weit verschoben, bis die eine Seite 22 des Objekttisches 6 entlang der Seite der Halterung 4 bzw. der Schmalseite der Kassette 5 liegt. Dies ist in der Fig. 3 dargestellt. Eine seitliche Verschiebung des Objekttisches 6 im Verhältnis zur Halterung 4 bzw. der Kassette 5 erfolgt, indem der Motor 14 die Schraube 12 so dreht, dass die mit einem Gewinde versehene Hülse 15 und dadurch der Objekttisch 6 in die Längsrichtung der Schraube 12 verschoben wird. Wenn die Hülse 20, die gleichzeitig entlang der Stange 16 verschoben wird, den Anschlage 18 erreicht hat, ist die gewünschte Verschiebung des Objekttisches 6 erfolgt. Nachdem die Kompressionsplatte 7 in dieser Seitenlage die Brust zusammengepresst hat, kann eine Aufnahme erfolgen.

In Verbindung mit einer seitlichen Aufnahme von der rechten Achselhöhle des Patienten wird der Objekttisch 6 im Verhältnis zur Halterung 4 in eine Lage verschoben, in der die andere Seite 23 des Objekttisches 6 entlang der anderen Seite der Halterung 4 bzw. der anderen Schmalseite der Kassette 5 liegt. Dies erfolgt, indem der Motor 14 nun die Schraube 12, im Vergleich zu dem, was bereits beschrieben worden ist, in die entgegengesetzte Richtung dreht, so dass die Hülse 15 und dadurch der Objekttisch verschoben wird, bis die Hülse 19, die entlang der Stange 16 läuft, den Anschlag 17 erreicht.

Eine derartige Lage des Objekttisches 6 ist in der Fig. 3 mit strichpunktierten Konturen desselben gezeigt.

Dadurch, dass sich die Halterung 4 bzw. die Kassette 5 in Verbindung mit den beschriebenen Bewegungen im Verhältnis zur Röntgenröhre 3 nicht bewegt haben, ist es auch nicht notwendig, mit Hilfe der vorhandenen Röntgenblenden eine meistens zeitraubende Einstellung des Strahlenfeldes, das die Kassette 5 bzw. den Röntgenfilm decken soll, vorzunehmen.

Der in der Beschreibung erwähnte Motor 14 kann ein Schrittmotor sein, der beim Einschalten desselben nach Wunsch den Objekttisch 6 teils in eine Mittellage bzw. zentrierten Lage für eine vertikale Aufnahme und teils in eine äußere rechte oder eine äußere linke Lage im Verhältnis zur Halterung für seitliche Aufnahmen verschieben kann.

Im Rahmen der Erfindung kann die Verschiebung des Objekttisches 6 gegenüber der Halterung 4 auch manuell erfolgen, wobei der Objekttisch in den beschriebenen Lagen mit Hilfe bekannter und daher nicht näher beschriebener Verschlussvorrichtungen arretiert werden kann.

Der in der Anmeldung erwähnte Bildempfänger kann, ausser einer Röntgenfilmkassette auch eine Bild- oder CCD-Platte sein.

## Patentansprüche

1. Röntgendiagnostikgerät für Mammographieuntersuchungen mit einem Arm (2) für eine Röntgenröhre (3), einer Halterung (4) für einen Bildempfänger (5) und einem Objekttisch (6, wobei der Objekttisch (6) seitlich gegen die Halterung (4) verschiebbar ist, sowie eine zwischen der Röntgenröhre (3) und dem Objekttisch (6) angeordnete Kompressionsplatte (7), wobei der Arm (2) um eine in einem Stativ (1) angebrachte horizontale Welle (9) drehbar angeordnet und die Halterung (4) für den Bildempfänger (5) mit dem Arm (2) verbunden ist,
und der Objekttisch (6) so ausgebildet ist, dass er gegenüber der Halterung (4) und gegenüber dem Bildempfänger (5) aus einer Ausgangsposition seitlich in eine erste und eine zweite Endposition verschiebbar ist, wobei die Ausgangsposition derart ist, dass beide Kurzseiten des Bildempfängers (5) in gleicher Entfernung von den Seiten des Objekttisches angeordnet sind und die Endpositionen derart sind, dass jeweils eine Kurzseite des Bildempfängers (5) im wesentlichen mit der Seite des Objekttisches (6) abschließt, **dadurch gekennzeichnet, dass** die Kompressionsplatte mit dem Arm (2) verbunden und entlang diesem verschiebbar ist.

## Claims

1. X-ray diagnosis apparatus for mammography examinations having an arm (2) for an X-ray tube (3), a mounting assembly (4) for an image receiver (5) and a stage (6), whereby the stage (6) can be moved laterally against the mounting assembly (4), as well as a compression plate (7) arranged between the X-ray tube (3) and the stage (6), whereby the arm (2) is arranged so that it can be rotated about a horizontal shaft (9) fitted in a stand (1) and the mounting assembly (4) is connected to the arm (2) for the image receiver, and the stage (6) is designed such that it can be moved laterally from a starting position into a first and second end position towards the mounting assembly (4) and towards the image receiver (5), the starting position being such that both short sides of the image receiver (5) are arranged at an equal distance from the sides of the stage, and the end positions are such that a short side of the image receiver (5) essentially terminates at the side of the stage (6) in each instance, **characterised in that** the compression plate is connected to the arm (2), along which it can move.

## Revendications

1. Appareil de radiodiagnostic pour la mammographie comprenant un bras (2) pour un tube (3) radiogène, une fixation (4) pour un récepteur (5) d'image et une table objet (6), la table objet (6) pouvant coulisser latéralement par rapport à la fixation (4), ainsi qu'une plaque (7) de compression interposée entre le tube (3) radiogène et la table objet (6), le bras (2) étant monté tournant autour d'un arbre (9) horizontal monté dans un statif (1) et la fixation (4) pour le récepteur (5) d'image étant reliée à l'arbre (2) et la table objet (6) étant constituée de manière à pouvoir coulisser par rapport à la fixation (4) et par rapport au récepteur (5) d'image d'une position de départ latéralement dans une première et dans une deuxième position finales, la position de départ étant telle que les deux petits côtés du récepteur (5) d'image sont à la même distance des côtés de la table objet et les positions finales étant telles que respectivement un petit côté du récepteur (5) d'image est sensiblement aligné avec le côté de la table objet (6), **caractérisé en ce que** la plaque de compression est reliée au bras (2) et peut coulisser le long de celui-ci.
